# EUROPEAN PATENT SPECIFICATION

(11) **EP 0 402 468 B1**
(45) Date of publication and mention of the grant of the patent: **13.09.1995**
(21) Application number: 89902810.4
(22) Date of filing: 22.02.1989
(51) Int. Cl.: C12M 3/00, F04F 1/06, A01K 63/00, A01G 31/00

(54) **CONTINUOUS PERFUSION APPARATUS UTILIZING AERATION**
KONTINUIERLICHE PERFUSIONSVORRICHTUNG MIT LÜFTUNG
DISPOSITIF DE PERFUSION EN CONTINU A AERATION

(30) Priority: 01.03.1988 JP 49348/88
(43) Date of publication of application: 19.12.1990
(73) Proprietor: Yuugen Kaisha PARASIGHT, Chiba 280 (JP)
(72) Inventor: NOJIMA, Youko, Kagoshima 890 (JP); NOJIMA, Hisatake, Kagoshima-shi Kagoshima 890 (JP)
(74) Representative: Goddar, Heinz J., Dr.
(86) International application number: PCT/JP89/00180
(87) International publication number: WO 89/08141

(56) References cited:
- DE-A- 249 564
- GB-A- 2 032 792
- JP-A- 0 549 004
- JP-A-63 173 576
- US-A- 3 898 018

## Description

### TECHNICAL FIELD

The present invention relates to an apparatus for perfusion of a liquid utilizing aeration, defined in the preamble part of the patent claim.

Perfusion of a breeding liquid for breathing control and nutrition supply is essential for breeding of the life such as cell culture, hydroponics and breeding. An apparatus for perfusing a liquid of small capacity is normally or usually such that water is moved by a water-flow pump to perfuse the liquid. For perfusion of a liquid of large capacity such as several hundred tons or several thousand tons, a super-huge water-flow pump is required in order to eliminate a stagnant area where the liquid is not perfused. Actually, a plurality of water-flow pumps are installed to cope with the stagnation, or such operation as to completely replace the breeding liquid copes with the stagnation. The invention can cope with the automatic perfusion of the above-described liquid of small capacity only by simple aeration. It is considered that, in a principle aspect, the invention can cope with perfusion of the liquid of large capacity.

An apparatus having the features of the preamble part of the patent claim is disclosed in US-A 3,898,018. The hydraulically controlled pneumatic ejector for pumping liquids disclosed therein works discontinuously, since the liquid is pumped away.

It is the object of the invention to provide an appartus for perfusion of a liquid utilizing aeration which is capable to work continuously.

This object is solved by the apparatus of the patent claim.

The invention is originally directed to a perfusion apparatus, but, if a one-way-valve is installed, the invention functions as a water-flow-pump. First, the background of the water-flow-pump will be described. Mechanical construction of the normal or usual water-flow pump utilizes a driving force of a rotor which takes various configurations. Although it is possible to create the water-flow pump from combination of piston movement and the one-way-valve, appearance of an electric motor, which is superior in efficiency, does not so much create the water-flow pump at present. However, the invention resembles the piston movement. That is, the invention perfuses the liquid upwardly and downwardly by water flow which utilizes the piston movement of air, and the valve is associated with the water flow to form the water-flow pump.

Regarding the perfusion apparatus, water is moved by the existing water-flow pump for the perfusion of the liquid of small capacity, to stir the liquid thereby eliminating the stagnant area, so that the liquid is perfused. In the invention, since the liquid is moved upwardly and downwardly so as to be perfused, the liquid is perfused without any stagnant area. Appearance of the stagnant area is inevitable for the liquid of large capacity. The invention solves difficulty of perfusion by a simple construction.

An important point in doing the invention is the function of a small pipe. That is, at one time, water enters a lumen and serves as "water plug" so that air is accumulated. Subsequently, the water in the lumen is blown out to aerate the lumen so that the accumulated air is quickly open and is removed.

### DISCLOSURE OF THE INVENTION

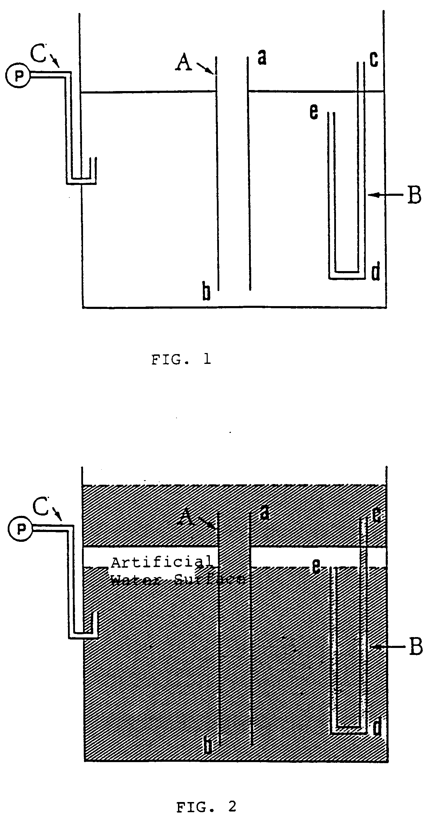

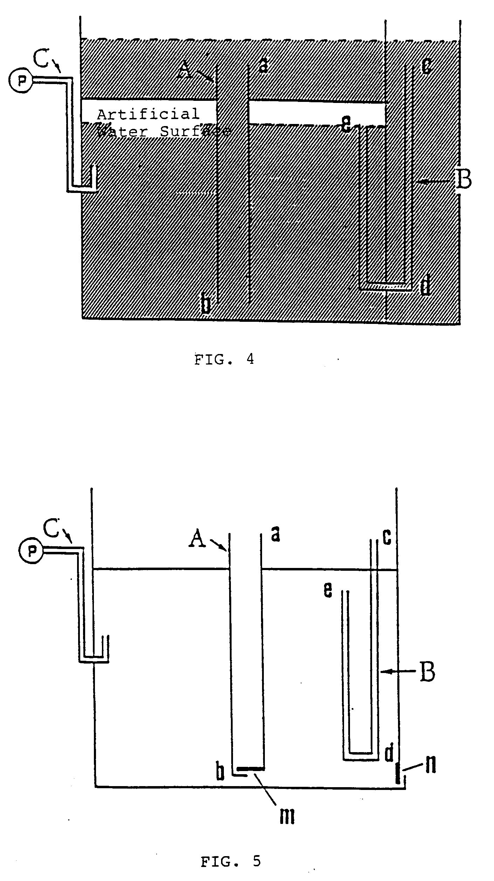

In common to Figs. 1 through 5, the arrows A, B and C indicate a tube, a small pipe and a blast pipe, respectively. Water has its surface which is covered with the shaded area. In common to Figs. 1 through 4, a and b denote an uppermost end and a lowermost end of the tube, respectively, while c, d and e designate an uppermost end on the outside of a container, a lowermost end on the inside of the container (also on the outside of the container in Fig. 4), and an uppermost end on the inside of the container, respectively.

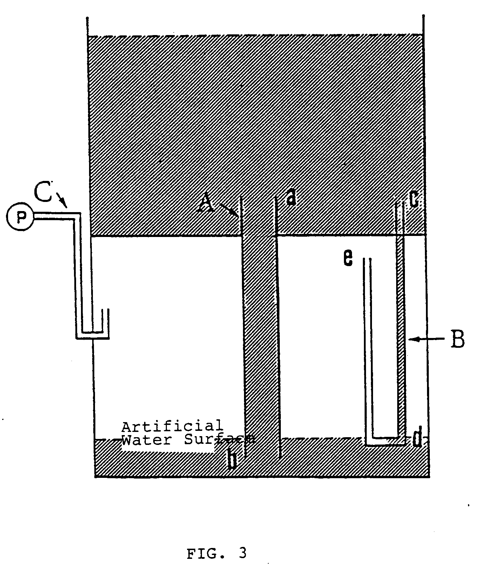

Fig. 1 is a vertical cross-sectional view of an apparatus in which no water enters. If water enters without blowing of air under the condition illustrated in Fig. 1, the water enters the container through the tube A, and creates an artificial water surface at the height of e, and the excess or surplus water is accumulated on an upper wall. At this time, the condition is brought to one illustrated in Fig. 2. Subsequently, air blowing starts through the blast pipe C, the artificial water surface descends until d that is the lowermost end of the pipe B. Simultaneously, the water surface within the pipe also descends. In due course, the artificial water surface (water surface within the pipe) further descends beyond d. This is represented in Fig. 3. Now, in the case where the pipe B is sufficiently thin in inner diameter, for example, of the order of 5 mm, the gravitational weight and the surface tension of the water act upon each other so that the water serving as the plug within the lumen of the pipe is discharged, and the lumen of the pipe is aerated. Thus, the air quickly escapes upwardly through the pipe. Outflow of the air completes at the height of e. Then, again, the water flows backward in the pipe so that the lumen of the pipe is plugged by the water. Thus, the pipe becomes interruption of communication. Since the air is blown continuously, the artificial water surface again starts to descend. In this connection, if the inner diameter of pipe B becomes in the order of 40 mm, the surface tension of the water tends to be nullified by the gravitational weight of the water, so that the water is not discharged and the pipe is not completely aerated. As a result, only a small quantity of delivered air ascends and the pipe is aerated. At this time, since the plug for the water cannot be released, there is no function of continuous perfusion.

The positions, where c, d and e in the small pipe B are located respectively, are very important. If c is not sufficiently higher than e, there is a fear that the water does not flow well backward. If c and e have their difference (water level difference) therebetween of the order of 1.5 cm, the water flows well backward. If the position of d is immediately above the horizontal level of the entrance b of the tube A, and if there is no difference (water level difference), there is such a possibility that the air passes through the tube. d and b should have their difference therebetween more than a value of the order of 1 cm.

It is not necessarily required that the opening c of the small pipe B is located on the upper wall of the container. As shown in Fig. 4, if an adequate water level is given by another container which is attached to the container, the apparatus has a function of perfusion.

There is particularly no limit or restriction as to the position of the blast pipe within the container. If the blast pipe is installed at a lower position, there is loss in energy. If the quantity of aeration is excessively large, timing of backward flow of the water (water level of e) is lost. In this case, the inner diameter of the pipe B should be enlarged.

In Fig. 5, m designates a valve which is mounted at b (lowermost end) of the tube A. The valve is a one-way-valve open only upwardly. n denotes a valve mounted to the side wall which is located at a lower section of the container. The valve opens one way only toward the interior of the container. Fig. 5 is one at the time the apparatus according to the invention has a function of a pump, and is a vertical cross-sectional view thereof.

### INDUSTRIAL APPLICABILITY

This invention is compelled to perfusion or, rotating or involving culture of the culture fluid in order to supply oxygen and nutrition in culture of animals or plant cells. The perfusion apparatus according to the invention is directed to a new and inexpensive method.

Further, if the one-way-valve is mounted to determine flow of the liquid in a constant direction, the perfusion apparatus has a function as the water-flow pump. This means that only air piping enables a simple and inexpensive pump to be installed which does not use electricity.

## Claims

1. Apparatus for perfusion of a liquid utilizing aeration, comprising an intermediate lid, mounted to a bucket to completely seal the latter, the bucket having a sealed container on its lower side, further comprising a hollow tube A extending through an upper wall of the container, an upper entrance a of the tube A being located above the upper wall on the outside of the container, while a lower entrance b is positioned adjacent a bottom wall at a location within the container, a small pipe B having a first opening c and a second opening e located at a position immediately below the upper wall at a location within the container, an intermediate section of the small pipe B being folded and bent downwardly such that a lowermost end d of the small pipe B is located above the lower entrance b of the tube A, and a blast pipe C for delivering gas such as air or the like, characterized in that the first opening c of that small pipe B which has an inner diametre of less than 40 mm, is located above the upper wall at a location on the outside of the container, the level difference between the openings c and e being in the order of 1,5 cm, and the level difference between the lowermost end d of the small pipe B and the lower entrance b of the tube A being more than about 1 cm and said blast pipe C extends through the container, so that, if the liquid enters the bucket through the tube A and if gas is continously delivered through the blast pipe C, the liquid practices such vertical movement that the liquid slowly ascends through the tube and quickly descends in order to participate in another cycle.

## Patentansprüche

1. Vorrichtung für die Perfusion einer Flüssigkeit unter Verwendung von Belüftung, mit einem zwischengeschalteten Deckel, der an einer Wanne angebracht ist, um die letztere vollständig abzudichten, wobei die Wanne einen abgedichteten Behälter auf ihrer unteren Seite hat, weiterhin mit einem hohlen Rohr A, das sich durch eine obere Wand des Behälters erstreckt, wobei ein oberer Einlaß a des Rohrs A oberhalb der oberen Wand auf der Außenseite des Behälters angeordnet ist, während ein unterer Einlaß b benachbart einer Bodenwand an einer Stelle innerhalb des Behälters positioniert ist, einem kleinen Rohr B, das eine erste Öffnung c und eine zweite Öffnung e an einer Position unmittelbar unterhalb der oberen Wand an einem Ort innerhalb des Behälters angeordnet hat, wobei ein Zwischenabschnitt des kleinen Rohres B abgeklappt und nach unten gekrümmt ist, so daß ein unterstes Ende d des kleinen Rohres B oberhalb des unteren Einlasses b des Rohres A angeordnet ist, und einem Lüftungsrohr C zum Liefern eines Gases, so wie Luft oder dergleichen, dadurch gekennzeichnet, daß die erste Öffnung c des kleinen Rohres B, das einen Innendurchmesser von weniger als 40 mm hat, oberhalb der oberen Wand an einer Stelle auf der Außenseite des Behälters angeordnet ist, wobei der Pegelunterschied zwischen den Öffnungen c und e in der Größenordnung von 1,5 cm ist und der Pegelunterschied zwischen dem untersten Ende d des kleinen Rohres B und dem unteren Einlaß b des Rohres A mehr als etwa 1 cm ist und das Lüftungsrohr C sich durch den Behälter erstreckt, so daß, wenn die Flüssigkeit in die Wanne durch das Rohr A eintritt und wenn Gas kontinuierlich durch das Lüftungsrohr C geliefert wird, die Flüssigkeit eine solche vertikale Bewegung ausführt, daß die Flüssigkeit langsam durch das Rohr aufsteigt und schnell absteigt, um in einem anderen Zyklus teilzunehmen.

## Revendications

1. Appareil pour la perfusion d'un liquide, utilisant l'aération, comprenant un couvercle intermédiaire monté sur une cuve pour la fermer complètement, la cuve ayant un récipient fermé hermétiquement sur son côté inférieur, comprenant en outre un tube creux A passant à travers une paroi supérieure du récipient, une entrée supérieure a du tube A étant située au-dessus de la paroi supérieure, à l'extérieur du récipient, tandis qu'une entrée inférieure b est placée adjacente à une paroi inférieure, en un emplacement dans le récipient, un petit tuyau B ayant une première ouverture c et une deuxième ouverture e située en une position immédiatement au-dessous de la paroi supérieure, en un emplacement dans le récipient, une section intermédiaire du petit tuyau B étant pliée et incurvée vers le bas, de manière que l'extrémité d la plus basse du petit tuyau B soit située au-dessus de l'entrée inférieure b du tube A, et un tuyau d'évent C pour fournir du gaz tel que de l'air ou analogue, caractérisé en ce que la première ouverture c de ce petit tuyau B qui a un diamètre intérieur inférieur à 40 mm est située au-dessus de la paroi supérieure, en un emplacement situé à l'extérieur du récipient, la différence de niveau entre les ouvertures c et e étant de l'ordre de 1,5 cm, et la différence de niveau entre l'extrémité d la plus basse du petit tuyau B et l'entrée inférieure b du tuyau A étant supérieure à à peu près 1 cm et ledit tuyau d'évent C traverse le récipient, de manière que, si le liquide entre dans la cuve via le tube A et si du gaz est fourni en continu par le tuyau d'évent C, le liquide effectue un mouvement vertical tel que le liquide monte lentement dans le tuyau et descend rapidement, en vue de participer à un autre cycle.
